Europäisches Patentamt

European Patent Office

Office européen des brevets

(19)

(11) Publication number: **0 295 959**
A2

(12)

# EUROPEAN PATENT APPLICATION

(21) Application number: 88305580.8

(22) Date of filing: 17.06.88

(51) Int. Cl.⁴: **C 12 N 15/00**
C 12 N 1/20, C 12 N 5/00,
A 01 H 1/00, C 12 P 21/02,
A 23 K 1/00

(30) Priority: 19.06.87 US 65303

(43) Date of publication of application:
21.12.88 Bulletin 88/51

(84) Designated Contracting States:
AT BE CH DE ES FR GB GR IT LI LU NL SE

(71) Applicant: PLANT CELL RESEARCH INSTITUTE, INC.
6560 Trinity Court
Dublin California 94568 (US)

(72) Inventor: Sun, Samuel, S.M.
2326 Lariat Lane
Walnut Creek California 94596 (US)

Altenbach, Susan B.
273 Marlow Drive
Oakland California 94605 (US)

(74) Representative: Bizley, Richard Edward et al
BOULT, WADE & TENNANT 27 Furnival Street
London EC4A 1PQ (GB)

(54) Sulphur-rich protein from bertholletia excelsa H.B.K.

(57) Methods and contructs are provided which allow for nutritional improvement of plants and plant tissue through inducing production of the sulfur-rich 2S seed storage protein from B. excelsa H.B.K.

EP 0 295 959 A2

Bundesdruckerei Berlin

## Description

## SULFUR-RICH PROTEIN FROM BERTHOLLETIA EXCELSA H.B.K.

The present invention is directed to plants and plant proteins. More particularly, the present invention is directed to sulfur-rich proteins from the plant Bertholletia excelsa H.B.K., and the nutritional enhancement of organisms, such as higher plants, with such protein.

The seeds of Bertholletia excelsa H.B.K. (Brazil nuts) are a major export from the Amazon Basin region. The seed storage proteins from Brazil nuts have unusually high levels of sulfur-containing amino acids (approximately 8.3% by weight) and are probably the richest food source of these essential amino acids. The total protein of Brazil nuts can be fractionated in three size classes of proteins: the 11S, 7S and 2S proteins. The 2S fraction, which is water-soluble and classified as albumin, comprises about 30% of the total protein and is exceptionally rich in sulfur amino acids; about 17% methionine and 13% cysteine. Youle & Huang, (1981) Am. J. Bot. 68:44-48.

Many vegetable proteins, especially those from legumes, are deficient in the essential sulfur amino acids, methionine and cysteine. Brazil nut protein can compensate for this deficiency. For example, defatted Brazil nut flour can be used to supplement the poor protein quality of navy beans. Antunes et al. (1977) J. Agric. Food Chem. 25:1096. Increased information about the sulfur-rich proteins of B. excelsa may provide an approach to improve the nutritive value of vegetable protein sources (as well as other sources) deficient in the sulfur amino acids.

Other seed storage proteins have been cloned and expressed in heterologous plants. For example, the seed storage protein from Phaseolus vulgaris (French bean) has been cloned and expressed in heterologous plants under the control of its own promoter and heterologous promoters, See, e.g., Murai et al. (1984) Science 222:476; Segupta-Gopalan et al. (1985) Proc. Natl. Acad. Sci, USA 82:3320; EPO Pub. No. 126,546; EPO Pub. No. 122,791.

The following publications may also be of interest: Sun et al. (1987) J. Agric. Food Chem. 35:232-235; Sun et al. (1987) Eur. J. Biochem. 162:477-483; Altenbach et al. (1987) Plant Mol. Biol. 8:239-250; Ampe et al. (1986) Eur. J. Biochem. 159:597-604; Castro et al. (1987) Mol. Gen. Genet. 206:338-343; Sun et al. (1983) J. Cell. Biol. 7B:278; Sun et al. (1984) Plant Phys. Supp. 75:64; Aldenbach et al. Id.; Sun et al. US-Australia Seed Protein Workshop (Feb. 11-15, 1985); Altenbach et al. (1985) J. Cell. Biochem. 9C:212; Altenbach et al. (1987) J. Cell. Biochem. 11B:49; Tao et al. (1987) Fed. Proc. 46:891.

The present invention provides for improving the nutritional value of edible organisms, including, but not limited to, higher plants. In particular, the present invention provides for the introduction of sulfur-rich proteins from the seeds of B. excelsa H.B.K. and nucleic acid sequences encoding these sulfur-rich proteins.

In one embodiment, the present invention provides nucleic acids, such as DNA molecules, which encode one or more subunits of the sulfur-rich 2S seed storage protein of B. excelsa H.B.K., including such sequences in expression cassettes. In addition, the present invention provides nucleic acid sequences which encode prosequences and preprosequences of the 2S seed storage protein.

In another embodiment, the present invention provides a cell comprising a replicon containing an expression cassette encoding at least one subunit of the sulfur-rich 2S seed storage protein of B. excelsa H.B.K. under the control of regulatory sequences that provide for the expression of said subunit in said cell, said subunit being heterologous to said cell. In particularly preferred embodiments, the cellular host is a higher plant cell.

In other aspects the invention provides the following:-

(a) A method of producing a DNA molecule comprising an expression cassette containing a coding sequence encoding at least one subunit of the sulfur-rich 2S seed storage protein of Bertholletia excelsa H.B.K., which method comprises ligating to said coding sequence regulatory sequences that provide for the transcription of said coding sequence in a cell, the promoter of said regulatory sequences being heterologous to said coding sequence and/or a signal sequence encoded by said coding sequence being heterologous to said subunit; and

(b) A method of making a cell having expressible DNA encoding at least one subunit of the sulfur-rich 2S seed storage protein of Bertholletia excelsa H.B.K., which method comprises (a) incorporating into the expressible genome of a cell DNA of the invention; or, (b) incorporating into the cell a DNA coding sequence encoding at least one subunit of the sulfur-rich 2S seed storage protein of Bertholletia excelsa H.B.K. under the control of DNA regulatory sequences that provide for the transcription of said coding sequence in said cell, said subunit being heterologous to said cell; and

(c) A process for the production of a plant containing the sulfur-rich 2S seed storage protein of Bertholletia excelsa H.B.K. comprising introducing into the expressible genome of selected plant tissue other than Bertholletia excelsa H.B.K., a DNA coding sequence encoding at least one subunit of the sulfur-rich 2S seed storage protein of Bertholletia excelsa H.B.K. under the control of DNA regulatory sequences that provide for the transcription of said coding sequence in cells of said tissue and culturing the resulting engineered tissue; and

(d) A foodstuff or foodstuff ingredient comprising tissue/cells other than Bertholletia excelsa H.B.K. enriched with a sulfur-rich 2S seed storage polypeptide of Bertholletia excelsa H.B.K.; and

(e) A process for providing a foodstuff or foodstuff ingredient as defined in Claim 18, which comprises engineering cells other than Bertholletia excelsa H.B.K. to express at least one subunit of the sulfur-rich 2S seed storage protein of Bertholletia excelsa H.B.K. and cultivating said cells.

Figure 1 shows the complete nucleic acid sequence of a cDNA which encodes an isoform of the sulfur-rich 2S seed storage protein from Brazil nut. The putative amino acid sequence is also shown, as well as the amino acid sequence determined from purified proteins.

Figure 2 shows the sequence of a genomic clone of the sulfur-rich Brazil nut storage protein, as well as a comparison with the cDNA sequence.

Figure 3 is a schematic representation of the processing of the sulfur-rich Brazil nut storage protein from the preprosequence to the two mature subunits.

Figures 4 and 5 are a schematic representation of the construction of the expression cassettes encoding sulfur-rich Brazil nut polypeptides.

Figure 6 shows (A) the partial amino acid sequence of the 9 kd subunit and (B) the probes used to clone cDNAs.

Figure 7 shows the construction of pARC12.

In addition to the techniques described below, the practice of the present invention will employ conventional techniques of molecular biology, microbiology, recombinant DNA technology, and plant science, all of which is within the skill of the art. Such techniques are explained fully in the literature. See, e.g., Maniatis et al., Molecular Cloning: A Laboratory Manual (1982); DNA Cloning: Volumes I and II (D.N. Glover ed. 1985); Oligonucleotide Synthesis (M.J. Gait ed. 1984); Nucleic Acid Hybridization (B.D. Hames & S.J. Higgins eds. 1985); Transcription and Translation (B.D. Hames & S.J. Higgins eds. 1984); Animal Cell Culture (R.I. Freshney ed. 1986; Plant Cell Culture (R.A. Dixon ed. 1985); Propagation of Higher Plants Through Tissue Culture (K.W. Hughes et al. eds. 1978; Cell Culture and Somatic Cell Genetics of Plants (I.K. Vasil ed. 1984); Fraley et al. (1986) CRC Critical Reviews in Plant Sciences 4:1 (hereinafter Plant Sciences); Biotechnology in Agricultural Chemistry: ACS Symposium Series 334 (LeBaron et al. eds. 1987).

In describing the present invention, the following terminology will be used in accordance with the definitions below.

A "replicon" is any genetic element (e.g., plasmid, cosmid, chromosome, virus, etc.) that behaves as an autonomous unit of DNA replication in vivo; i.e., capable of replication under its own control within a cell.

A "vector" is a replicon, such as a plasmid, cosmid, or bacteriophage, to which another DNA segment may be attached so as to bring about replication of the attached segment.

A "DNA molecule" refers to the polymeric form of deoxyribonucleotides (adenine, guanine, thymine, or cytosine) in either single- or double-stranded form.

When in the double-stranded form, the molecule will usually be in its normal, double-stranded helix. The term "DNA molecule" is not limited to any particular tertiary form of DNA. Thus, the term includes double-stranded DNA found, inter alia, in linear DNA molecules, viruses, plasmids, and chromosomes. When discussing the structure of particular double-stranded DNA molecules, sequences may be described herein according to the normal convention of giving only the sequence in the 5′ to 3′ direction (left to right) along the nontranscribed (anti-sense) strand of DNA (i.e., the strand having a sequence homologous to the mRNA). If both strands, are shown, the anti-sense strand will be on top.

A DNA "coding sequence" is a DNA sequence which is transcribed and translated into a polypeptide in vivo when placed under the control of appropriate regulatory sequences. The boundaries of the coding sequence are determined by the start codon at the 5′ terminus and a translation stop codon at the 3′ terminus. Examples of coding sequences include cDNA reverse transcribed from eukaryotic mRNA, genomic DNA sequences from eukaryotic cells, and synthetic DNA sequences.

A cell which has been "transformed" by an exogenous DNA sequence is a cell into which the exogenous DNA has been introduced. The exogenous DNA may be integrated (covalently linked) to chromosomal DNA making up the genome of the cell, or it may remain extrachromosomal. "Stably" integrated DNA sequences are those which are inherited through chromosome replication by daughter cells or organisms (accounting for loss by Mendelian segregation). This stability is exhibited by the ability to establish cell lines or clones comprised of a population containing the exogenous DNA.

A "clone" or "cell line" is a population of cells descended from a single cell or common ancestor by mitosis and is capable of stable growth in vitro for many generations.

A composition of a first type of matter (e.g., a DNA molecule containing a coding region) is "substantially free" of a second type of matter (e.g., DNA molecules which do not contain a coding region) if the composition is comprised of less than about 10% (weight/weight) of the second type of matter relative to the sum of the first and second types of matter. Preferably, the composition contains less than about 5% of the second type of matter, most preferably less than about 1%.

The term "2S seed storage protein of Bertholletia excelsa H.B.K." refers to the art-recognized class of polypeptides comprised of highly homologous isoproteins isolated from Brazil nuts. The isoforms of this protein class are characterized in that they: (i) constitute the major portion of the water-soluble albumins from Brazil nuts having a sedimentation coefficient of 2S, (ii) contain high levels of cysteine and particularly high levels of methionine relative to other plant storage proteins, (iii) are encoded as a 17 kd preproprotein [18 kd by gel electrophoresis] which is in turn processed in vivo to 15 kd and 12 kd propeptides (Figure 3), and (iv) in their mature form, are comprised of a dimer of 3 kd and 9 kd subunits produced by proteolytic processing of the preproprotein. A representative isoprotein cDNA is shown in Figure 1, as well as putative amino acid sequences and the sequence of a native peptide. Figure 2 shows the nucleic acid sequence of a genomic clone corresponding to the cDNA of a second isoform, as well as its putative amino acid sequence. Other isoforms will be at least about 80% homologous at the amino acid sequence level to these representative members, preferably at least about 85% homologous, and more preferably at least about 90% homologous.

The expression of the 2S seed storage protein in vivo is shown schematically in Figure 3. The 2S seed storage protein is expressed initially as a 17 kd prepropeptide of approximately 146 amino acids in length. Amino acid residues 1 through about 22 appear to be a signal sequence. Proteolytic processing cleaves this presequence to provide a prosequence of about 15 kd comprising amino acid residues from approximately 23 through 146. This propeptide is then processed to a second propeptide of about 12 kd, constituting approximately amino acid residues 47-146. This second precursor polypeptide is then processed into the 3 kd and 9 kd subunits, which together form the dimeric mature molecule linked through disulfide bridges. Some data indicate that the 3 kd subunit is comprised of the sequence from about amino acid residue 47 through about amino acid residue 69 or 72. Other data shows the 3 kd subunit is comprised of amino acid residues 44 through 64. The 9 kd subunit is comprised approximately of amino acid residues 70 or 73 through 146.

In accordance with the present invention, a DNA molecule is provided which contains a coding region that encodes the sulfur-rich 2S seed storage protein of B. excelsa H.B.K. The DNA molecules encode one or both of the seed storage protein's subunits; i.e., the 3 kd and/or 9 kd subunits. For example, DNA molecules according to the present invention may contain a coding region which encodes just the 3 kd subunit, or just the 9 kd subunit. Alternatively, a DNA molecule according to the present invention can encode the 12 kd prosequence, which contains both the 3 kd and 9 kd subunits, or a fusion protein comprised of the 3 kd and/or 9 kd subunits. In yet other embodiments of the present invention, the DNA molecules could encode the 15 kd prosequence, or the 17 kd prosequence. DNA molecules according to the present invention can be comprised of a coding region which is entirely homologous to native DNA; e.g., cDNA or genomic DNA. Alternatively, the coding region can be partially or completely synthetic in nature; e.g., comprised of codons different than that found in the native plant, yet encoding substantially the same protein. This may be particularly preferred when it is intended to express the coding region in a heterologous host, thereby allowing for the selection of host-preferred codons. See, e.g., U.S. Pat. No. 4,356,270; EPO Pub. No. 46,039. The coding region may also contain sequences heterologous to the B. excelsa sequences. For example, it may be desirable to link the storage proteins of sequences to a heterologous signal sequence in place of the native signal peptide.

DNA molecules encoding Brazil nut 2S storage protein can be prepared synthetically by known methods of oligonucleotide synthesis. Synthetic coding sequences can be prepared from overlapping oligonucleotides whose sequences contain codons for the amino acid sequence of the storage protein. Such oligonucleotides are prepared by standard methods and assembled into a complete coding sequence. See, e.g., Edge (1981) Nature 291:756; Nambiar et al. (1984) Science 323:1299; Jay et al. (1984) J. Biol. Chem. 259:6311; Oligonucleotide Synthesis (supra).

Brazil nut storage protein coding sequences can also be isolated employing methods of screening cDNA or genomic libraries. While probes can be prepared from the nucleic acid sequences disclosed herein, it is preferred to employ the entire coding sequences disclosed herein as a probe when screening libraries for other isoforms of the Brazil nut 2S storage protein. Due to the high degree of homology between the various isoforms, isolation of additional DNA sequences encoding different isoproteins is within the skill of the art by employing the nucleic acid sequences disclosed herein as probes. See, e.g., Molecular Cloning: A Laboratory Manual (supra); DNA Cloning: Vol. I & II (supra); Nucleic Acid Hybridization (supra).

The synthesis or isolation of DNA molecules containing the described coding sequences permits those of skill in the art to prepare compositions of DNA molecules encoding Brazil nut storage protein substantially free of DNA molecules which do not encode the storage protein. The compositions of DNA molecules containing coding sequence according to the present invention can be comprised of linear coding sequences, or coding sequences in cloning vectors. Numerous cloning vectors are known to those skilled in the art, and the selection of an appropriate cloning vector is a matter of choice. Examples of recombinant DNA vectors for cloning in the host cells which they transform include bacteriophage lambda (E. coli), pBR322 (E. coli), pACYC177 (E. coli), pKT230 (gram-negative bacteria), pGV1106 (gram-negative bacteria, pLAFR1 (gram-negative bacteria), pME290 (non-E. coli gram-negative bacteria), pHV14 (E. coli and Bacillus subtilis), pBD9 (bacillus), pIJ61 (Streptomyces), pUC6 (Streptomyces), actinophage phi C31 (Streptomyces), YIp5 (yeast), YCp19 (yeast), and bovine papilloma virus (mammalian cells). See, generally, DNA Cloning: Volumes I & II (supra); Molecular Cloning: A Laboratory Manual (supra).

In accordance with the present invention, DNA constructs are provided in which the above-described coding sequences are placed under the control of transcription regulatory sequences so that the Brazil nut 2S storage protein is expressed in a heterologous host cell. Such DNA constructs, referred to as "expression cassettes", are comprised of the storage protein coding sequence under the control of regulatory sequences, such as a promoter, ribosome binding site (for bacterial expression) and a transcription termination sequence or polyadenylation signal. Other regulatory sequences include operators, enhancers, and the like. The cassette is usually flanked by convenient restriction sites. The selection of the appropriate regulatory sequence and coding sequence, as well as their assembly for expression in a particular host, is within the skill of the art.

It is usually desirable to include within the expression cassette a selectable marker. A "selectable marker" gene encodes a "selectable phenotype"; i.e., a phenotype of a cell or organism which allows for the identification or selection of cells expressing the selectable marker gene. Well-known marker genes are known in the art, including, but not limited to, the gene for chloramphenicol acetyltransferase (CAT), neomycin phosphotransferase (neo$^R$), neomycin phosphotransferase II (npt-II), nopaline synthase (NOS), hygromycin phosphotransferase, the glyphosate resistance gene (EPSP), dihydrofolate reductase (mtx$^R$), hypoxanthine phosphoribosyltransferase (hpt), and thymidine kinase (tk). Cells transformed with sequences encoding these proteins are able to survive on media which would otherwise be toxic to the cell. Other types of markers, such as beta-galactosidase (lacZ) cause cells transformed therewith to change color under certain conditions, thus allowing for visual selection. The presence of the selectable marker in the expression cassette allows for the determination of whether a particular cell has been transformed stably by the expression cassette. Selectable markers can be included which function in the ultimate host which will express the storage protein, as well as additional markers which will function in intermediate hosts in which the construction of the expression cassette occurs.

The expression cassette is constructed so that the coding sequence is located within the cassette with the appropriate control sequences, the positioning and orientation of the coding sequence with respect to the control sequences being such that the coding sequence can be transcribed under the control of the regulatory sequences (i.e., by RNA polymerase which attached to the DNA molecule at the control sequences) in a host cell transformed by the expression cassette. It is possible to assemble the expression cassette prior to inserting it into a cloning vector as described above. Alternatively, an expression cassette can be constructed by cloning the coding sequence directly into an expression vector already containing the appropriate regulatory sequences and a restriction site downstream from the promoter.

Construction of expression vectors or expression cassettes for use in transforming microbial hosts (e.g., bacteria or yeast) may be desired to produce single-cell protein by fermentation to supplement the nutritive value of other protein sources. A number of prokaryotic expression vectors are known in the art which could be adapted for this purpose See, e.g., U.S. Patent Nos. 4,440,859; 4,436,815; 4,431,740; 4,431,739; 4,428,941; 4,425,437; 4,418,149; 4,411,994; 4,366,246; 4,342,832. See also British Patent Nos. 2,121,054; 2,008,123; 2,007,675; and European Patent Pub. No. 103,395. Yeast expression vectors are also known in the art. See, e.g., U.S. Patent Nos. 4,446,235; 4,443,539; 4,430,428; 4,546,082. See also European Patent Pub. Nos. 103,409; 100,561; 96,491.

A preferred class of heterologous hosts for the expression cassettes containing coding regions for the Brazil nut 2S storage protein are eukaryotic hosts, particularly the cells of higher plants (dicots, monocots). Particularly preferred among the higher plants are those of agronomic value having edible parts low in sulfur-containing proteins, such as leguminous plants, including, but not limited to, Glycine max (soybean), Medicago sativa (alfalfa), Psophocarpus tetragonolobus (winged bean), and Vigna aconitifolia (moth bean).

Expression cassettes intended for use in higher plants will employ regulatory sequences functional in such plants. For example, promoters can be selected from the group consisting of plant promoters, plant virus promoters, and T-DNA promoters (from both Ti and Ri plasmids). Specific T-DNA promoters known in the art are the nopaline synthase (NOS) promoter, and the octopine synthase (OCS) promoter. These promoters are constitutive promoters. See, e.g., Plant Sciences (supra). Examples of plant virus promoters include the 19S and 35S transcript promoters from cauliflower mosaic virus (CaMV. See, e.g., Id.; Koziel et al. (1984) J. Mol. Appl. Genet. 2:549. Numerous plant promoters have been shown to work in heterologous systems, including, but not limited to, the pea small subunit RUBP carboxylase (pSS) promoter, Morelli et al. (1985) Nature (London) 315:200; Broglie et al. (1984) Science 224:838; Herrera-Estrella et al. (1984) Nature (London) 310:115; Coruzzi et al. (1984) EMBO J. 3:1671; the soybean small subunit RUBP carboxylase (SbSS) promoter, Facciotti et al. (1985) Bio/Technology 3:241; the maize zein promoter, Matzke et al. (1984) EMBO J. 3: 1525; the wheat chlorophyll a/b binding protein promoter, Lampa et al. (1986) Nature (London) 316:750-752; soybean 7S alpha' conglycinin promoter, Beachy et al. (1985) EMBO J. 4:3047; the soybean glycinin G2 promoter; soybean heat-shock promoter, EPO Publication No. 159,884; and the french bean phaseolin promoter, Sengupta-Gopalan et al. (1985) Proc. Natl. Acad. Sci. USA 82:3320; Murai et al. (1984) Science 222:476. Depending upon the application, it may be desirable to select from among the available promoters those which are tissue specific (e.g., seed, leaf, etc.) and/or regulated (e.g., light-induced, temperature or heat-induced, developmentally regulated, etc.).

Particularly preferred promoters are those which allow for expression of the Brazil nut 2S storage protein in seeds of heterologous plants. Examples of such promoters include, but are not limited to, the phaseolin promoter and the soybean 7S alpha' conglycinin promoter. Expression cassettes intended for seed-specific expression can employ either heterologous promoters such as these, or the homologous 2S storage protein promoter. In like manner, expression cassettes can employ heterologous signal peptides in a coding sequence, particularly those associated with the heterologous promoter, or the 2S protein's own signal peptide.

Various methods of transforming cells of higher plants with expression cassettes according to the present invention are available to those skilled in the art. Among the most popular of transformation methods are those based on transformation vectors constructed of T-DNA from Ti or Ri plasmids. It is particularly preferred to use binary T-DNA vectors. See, e.g., Plant Sciences (supra). T-DNA based vectors have been shown to transform dicots and monocots, including, but not limited to, legumes (e.g., soybean, peas, and alfalfa), cotton, rape, tomato, Liliaceae (e.g., asparagus), Amaryllidaceae, etc. See, e.g., Plant Sciences (supra) and references sited therein; Pacciotti et al. (1985) Bio/Technology 3:241; Byrne et al. (1987) Plant Cell, Tissue and Organ Culture 8:3; Sukhapinda et al. (1987) Plants Mol. Bio. 8:209-216; Lorz et al. (1984) Proceedings of EEC-Symp.: In Vitro Techniques-Propagation and Long-Term Storage; Lorz et al. (1985) Mol. Gen. Genet. 199:178; Potrykus et al. (1985) Mol. Gen. Genet. 199:183.

Other transformation methods are available to those skilled in the art. Viral transformation vectors are known, such as those based on CaMV. See, e.g., Brisson et al. (1984) Nature (London) 310:511; Gronenborn et al. (1981) Nature (London) 294:773. Transposons can also be used to transform plants, particularly monocots. Maize transposons include, for example, Ac and Ds transposons, as well as the Mul transposon. See, e.g., Plant Sciences (supra) at 28.

In the absence of a suitable transformation vector for desired higher plant host, such cells can be transformed by the direct uptake of DNA including expression cassettes of the present invention. The uptake of foreign DNA in plant cells using various chemical agents is known. See, e.g., Davey et al. (1980) Plant Sci. Lett. 18:307; Draper et al. (1982) Plant Cell Physiol. 23:1; Krens et al. (1982) Nature (London) 296:72; Hain et al. (1985) Mol. Gen. Genet. 199:161; Hooykaas-van Slogteren et al. (1984) Nature (London) 311:763; Hernalsteens et al. (1985) EMBO J. 3:3039. Higher plant protoplasts have also been transformed by techniques of electroporation. See, e.g., Fromm et al (1986) Nature (London) 319:791; Fromm et al. (1985) Proc. Natl. Acad. Sci. USA 82:5824; Potter et al. (1984) Proc. Natl. Acad. Sci. USA 81:7161. Plant cells may also be transformed with foreign DNA by micro-injection using known techniques, or by high-velocity metal particles coated with RNA or DNA. See, e.g., Kline, Wolf, Wu, & Sanford, (1987) Nature (London) 327:70.

Once transformed plant cells have been produced, it is usually desirable to grow transformed cells into callus tissue or a cell line in suspension. Techniques for regenerating callus and for producing cell lines are known in the art. See, e.g., Plant Cell Culture (supra); Propagation of Higher Plants Through Tissue Culture (supra). Transformed cells can also be induced to undergo organogenesis. Thus, transformed cells can be maintained as undifferentiated tissues, or in organ culture. These plant cell cultures or organ cultures can be used, therefore, to produce the Brazil 2S nut storage protein. See, e.g., Flores in Biotechnology and Agricultural Chemistry; ASC Symposium Series 334, p.66 (LeBaron et al. eds., 1987).

Another important application of the present invention is the production of plants transformed with the coding sequences for the 2S Brazil nut storage protein. Techniques for regenerating plants from tissue culture, such as transformed protoplasts or callus cell lines, are known in the art. See, e.g., Bingham et al. (1975) Crop Sci. 15:719-721; Kao et al. (1980) Z. Pflanzenphysiol Bd. 96:135-141; Reisch et al. (1980) Plant Sci. Lett. 20:71-77; U.S. Pat. No. 4,548,901; Wright et al. (1987) Plant Cell Rpts. 6:83; Christianson et al. (1983) Science 222:632; Hammatt et al. (1987) Plant Science 48:129; Ghazi et al. (1986) Plant Cell Rpts. 5:452; Barwale et al. (1986) Planta 167:473; Newell et al. (1985) Plant Cell Tissue Organ Culture 4:145; Phillips et al. (1981) Plant Cell Tissue Organ Culture 1:123; Eapen et al. (1986) Theor. Appl. Genet. 72:384; Krishnamurthy et al. (1984) Plant Cell Rpts. 3:30; Shekhawat et al. (1983) Plant Sci. Lett. 32:43; Wilson et al. (1985) Plant Sci. 41:61; Venstesnaran et al. (1985) In Vitro 21(3,II): 36A. The selection of an appropriate method is within the skill of the art.

Set forth below are specific examples of the present invention which are intended for illustrative purposes only. These examples are not intended to limit the present invention in any manner. The references cited herein are incorporated by reference.

### EXAMPLES

### I. Cloning of Brazil Nut 2S Protein cDNA

Brazil nut fruits were obtained approximately 9 months after flowering from Brazil (Manaus) or Peru (Iquitos or Puerto Maldonaldo).

Brazil nut embryos were ground into a fine paste and defatted by extraction with hexane. The resulting defatted Brazil nut flour was then extracted in a buffer containing 1 M NaCl in 0.035 M sodium phosphate buffer, pH 7.5. The sulfur-rich protein was purified from this crude extract by the procedure of Youle and Huang, (1981) Amer. J. Bot. 68:44-48. The resulting sucrose gradient fractions were dialyzed extensively against deionized water at 4°C to precipitate the contaminating globulin proteins. The final protein sample contained polypeptides of 9 kd and 3 kd when analyzed on SDS-20% polyacrylamide gels.

The protein sample for sequencing was prepared by incubation of 2 mg of the purified sulfur-rich protein with 1 M Tris-HCl buffer, pH 8.8, containing 1 mM EDTA and 0.15 M 2-mercaptoethanol at 37°C under nitrogen gas for 4.5 hours. At the end of the incubation, iodoacetic acid was added to a final concentration of 0.22 M and the sample was incubated at 37°C in the dark for 30 minutes. After this treatment, the protein sample was dialyzed extensively against deionized water and lyophilized.

Sequence analysis of the sulfur-rich protein was performed by automated Edman degradation [Edman et al. (1967) Eur. J. Biochem. 1:80-90] on a Beckman 890°C liquid-phase sequenator equipped with a cold trap using program 050783 with 0.1 M Quadrol (Beckman Instruments, Inc.) and polybrene (2 mg) as a carrier. About 10 nmol of the sulfur-rich protein was applied into the liquid phase sequenator. Norleucine was added to the fractions and used as an internal standard for quantitation of each cycle. Phenylthiohydantoin-amino acids were identified and measured by (Packard 419) gas liquid chromatography [(1972) Anal. Biochem. 45:43-52], (Water 6000A) high performance liquid chromatography [(1978) J. Chromatogr. 148:532-535] and thin layer chromatography [(1974) Anal. Biochem. 59:564-573]. At least two of these methods were used at each step. A total of 60 cycles of degradation were conducted, and 97% repetitive yield was observed. No PTH-amino acid could be identified after cycle 57.

Two amino acid sequences were obtained from the analysis of the carboxymethylated sulfur-rich protein; one major (80%) and one minor (20%). The major sequence starts with Pro-Arg-Arg-Gly-Met... as $NH_2$-terminal amino acids, while the minor one start with Gly-Met... (Fig. 6A). The two protein sequences are identical in the region sequenced except that the minor one is three amino acids shorter than the major one at the $NH_2$ terminus; thus it was possible to determine the first 57 amino acids for the minor one. The sulfur-rich protein consists of two subunits, a 9 kd polypeptide and a 3 kd polypeptide. Both the 54 and the 57 amino acid sequences exceed the length of the 3 kd polypeptide, thus these sequences must represent 9 kd polypeptides, possibly two members of the 9 kd polypeptide family. An amino acid sequence for the 3 kd polypeptide was not obtained, suggesting that either the 3 kd polypeptide sequence is identical to the 9 kd sequence or the $HN_2$ terminus of the 3 kd polypeptide is blocked.

This amino acid sequence represents about 77% of the 9 kd subunit. The sequence contains unusually high levels of the sulfur amino acids: 21% methionine and 7% cysteine. There are two regions in the partial amino acid sequence where methionine residues are clustered with arginine residues: residues #29-35 (Arg-Met-Met-Met-Arg- Met-Met) and residues #47-52 (Met-Arg-Arg-Met-Met-Arg) (Fig. 6A)

An oligodeoxynucleotide probe was synthesized (by Biosearch, Inc.) which was complementary to the 6 possible RNA sequences encoding one of these methionine-rich regions (amino acid residues #30-35) (Fig. 6B). Polyadenylated RNA was prepared from 9-month old developing Brazil nut seeds by methods described previously for Phaseolus vulgaris [Hall et al. (1978) Proc. Natl. Acad. Sci. USA 75:3196-3200] and was cloned in the dimer-primer vector pARC7. Alexander et al. (1984) Gene 31:79-89. The resulting clones were screened by colony hybridization [Taub et al. (1982) Anal. Biochem. 126:222-230] using the above 5' labelled probe. The probe was hybridized to the filters in 6x NET (0.9 M NaCl, 0.09 M Tris/Cl, pH 7.5, 0.006 M EDTA), 0.05% NP-40, and 250 ug/ml yeast tRNA at 37°C for 20 hours. The filters were washed in 6x SSC at 37°C before autoradiography. Twelve clones were identified with inserts of 350 bp to 700 bp and further analyzed.

The cDNA sequence of one of the clones, pHS-3, was determined from both DNA strands by the dideoxy chain termination method. Sanger et al. (1977) Proc. Natl. Acad. Sci. USA 74:5463-5467. Where necessary regions of the clone were also sequenced by the method of Maxam and Gilbert, (1977) Proc. Natl. Acad. Sci. USA 74:560-564. The 25 nucleotides at the 5' end of the mRNA encoding the sulfur-rich protein were not represented in pHS-3 but were obtained by using a synthetic oligodeoxynucleotide complementary to nucleotides #49-69 as a primer to synthesize DNA complementary to the 5' end of the mRNA and sequencing the resulting extension product. For primer extension, the oligodeoxynucleotide 5' -AATCTTCGCCATGGTGATTCT-3', labelled at its 5' end, was annealed to 3 mg of poly(A)$^+$ RNA from the seeds of 9-month-old Brazil nuts in 8 mM Tris pH 7.5, 5 mM EDTA at 90°C followed by 15 minutes at 25°C. The annealed DNA sample was brought to a final concentration of 50 mM Tris pH 8.3, 5 mM DTT, 15 mM $MgCl_2$, 0.5 mM dNTPs, and 0.1 mg/ml BSA. AMV reverse transcriptase (BRL,. 37.5 units) was added and the reaction was incubated at 37°C for 90 minutes. EDTA was added to 20 mM and the sample was extracted twice with phenol:chloroform:isoamyl alcohol (25:24:1) and precipitated with ethanol. After denaturation, the samples were subjected to electrophoresis on an 8% sequencing gel. Three bands resulted which differed in length by single nucleotides. DNA from each of the three bands was eluted from the gel and sequenced by the method of Maxam and Gilbert.

Sequence analysis of pHS-3, demonstrates unequivocally that this cDNA encodes a polypeptide which is extremely rich in the sulfur-containing amino acids (Fig. 1). The sequence of pHS-3 is 599 nucleotides long excluding the poly(A) tail. By primer extension analysis using a 21 base synthetic oligodeoxynucleotide complementary to a region near the 5' end of pHS-3, it was determined that this cDNA clone falls 25 nucleotides short of the 5' end of the mRNA encoding the sulfur-rich protein. There are no ATG codons in the sequence of the primer extension product; thus, the first ATG codon found in pHS-3 (residues #58-60) represents the initiation codon for protein synthesis. This ATG fits with the consensus sequence for eucaryotic protein initiation sites. Kozak (1983) Microbiol. Rev. 47:1-45. A stop codon, TGA, is encoded by nucleotides #495-497 in pHS-3. The resulting open reading frame could encode a polypeptide of 146 amino acids, of which over 20% are sulfur-containing amino acids; 15.1% of these residues are methionine while 5.5% are cysteine. The first portion of the encoded polypeptide contains a large proportion of hydrophobic residues; of the 22 residues at the amino terminus of the protein, 36% are alanine and 18% are leucine. In comparison, the rest of the polypeptide is rich in arginine, glutamine and glutamic acid, a composition which is characteristic of other plant seed storage proteins. A hydropathy plot demonstrates that the amino terminus of the polypeptide is hydrophobic while the remainder of the polypeptide is largely hydrophilic.

By aligning the amino acid sequence derived from the nucleotide sequence with the major sequence determined from the purified 9 kd subunit, it was found that the coding region for the 9 kd polypeptide begins 265 nucleotides from the 5' end of the mRNA. By adding up the molecular weights of the individual amino acids encoded by this region, a value of almost 9 kd is reached. The amino acid sequence derived from the nucleotide sequence of the portion of the open reading frame between nucleotides 265 and 435 agrees quite well, although not precisely, with the major 57 residue partial amino acid sequence of the 9 kd subunit of the sulfur-rich protein (Fig. 1). Methionine residues are very predominant in the 9 kd subunit of the mature protein. There are 14 methionine residues in this region, representing 18.2% of the 77 amino acid polypeptide. Eight of these 14 methionines are found clustered with arginine residues in two regions of the polypeptide. In the first cluster, between amino acid residues #99 and 104, five out of six residues are methionines and four of the methionine residues are contiguous. The second methionine cluster, between amino acid residues #116 and 121, includes three methionine residues and three arginine residues. Interestingly, 2 of the 4 amino acid differences which are found between the amino acid sequence determined from the protein and that derived from the nucleotide sequence are found in the methionine-rich region that was used as a basis for the synthetic oligodeoxynucleotide probe. A second cDNA clone selected by the same probe was perfectly homologous with one of the sequences represented in the probe, suggesting that the sulfur-rich protein is encoded by a family of genes with some variation in these methionine-rich regions. The 9 kd subunit of the Brazil nut protein also contains a high proportion of cysteine (7.7%).

By hybrid-selected translation, it was found that pHS-3 is able to select a mRNA from a population of 9-month-old Brazil nut RNAs which directs the synthesis of an 18 kd polypeptide in vitro. This 18 kd polypeptide is immunoprecipitable with a polyclonal antibody raised in rabbits against the purified Brazil nut sulfur-rich protein, demonstrating conclusively that the sulfur-rich protein is synthesized initially as a large precursor polypeptide. See also Altenbach et al. (1987) Plant Mol. Biol. 8:239-250, the disclosure of which is incorporated herein by reference.

## II. Genomic Clone of Brazil Nut 2S Protein

Genomic DNA was isolated from embryos of fresh Brazil fruits by methods described previously for French bean [Sun et al. (1981) Nature 280:37]. The construction of a genomic DNA library of 1.2 x 10⁶ Charon 4A bacteriophage recombinants containing partial EcoRI fragments of Brazil nut DNA was as described by Blattner et al. J(1978) Science 202:1279, and Slightom et al. (1980) Cell 21:627]. This original library was amplified to 4.5 x 10¹⁰ recombinant phages and approximately 1.2 x 10⁶ of these were screened using a ³²P-labeled Brazil nut sulfur-rich protein cDNA (pHS-3) probe. A number of clones were identified which contained the sulfur-rich protein gene sequences. Figure 2 shows the nucleotide sequence of one genomic clone (Ch4A BN-7.2.1.1) of Brazil nut 2S protein.

## III. Construction of the Phaseolin-Brazil Nut 17 kd Chimeric Gene

A construct containing an expression cassette for the Brazil nut storage protein was prepared as described below. The construction is shown schematically in Figure 4.

A 3.8 kb BglII/BamHI fragment from phaseolin genomic clone Ch4A-177.4 [Sun et al. (1981) Nature (London) 280:37] was subcloned into the BamHI site of the plasmid pBR322, resulting in the clone pPh3.8. A 4 kb Sall-BamHI fragment of pPh3.8, which contains the phaseolin sequence as well as a small portion of pBR322, was inserted into the vector pTZ19U (Pharmacia, Analects, 13:4), resulting in the clone pSB-9. Site-directed mutagenesis was used to change a G residue to an A residue, thereby adding an AccI site four nucleotides upstream from the phaseolin termination codon. For the mutagenesis, single-stranded DNA prepared from pSB-9 was annealed to the 21-base oligonucleotide 5' -TATTCAGTATACAAATGCACC-3' in 0.02 M Tris, pH 7.5, 0.01 MgCl₂, 0.01 M DTT for 5 min at 80°C. The annealing mixture was then incubated at room temperature for 15 min. To 10 ul of the annealing mix was added 3 ul of 10X Buffer B (200 mM Tris pH 7.5, 100 mM MgCl₂, 40 mM DTT), 1.5 ul of 10 mM ATP, 1 ul of 2 mM dNTPs, 22.5 ul H₂O, 3 units of DNA ligase and 3 units of the Klenow fragment of PolI. The mixture was incubated at 30°C for 1 hour. An additional 1 ul of Buffer B, 1 ul of 2 mM dNTPs, 6 ul H₂O, 3 units of DNA ligase and 3 units of the Klenow fragment of PolI were then added and the mixture was incubated for an additional 3 hours at 30°C. The mutagenesis mixture (1 ul) was used to transform NM522 cells (Gough et al. (1983) M. Mol. Biol. 166:1-19. One clone, pB-13, was selected which contained the additional AccI restriction site.

Site-directed mutagenesis using single-stranded DNA prepared from pB-13 and the 21-base oligonucleotide 5'-CTCATCATAGTAGACTAGTAT-3' was then used to generate the plasmid pD-3 in which a C residue was changed to a G residue, thereby adding an AccI site five nucleotides upstream from the phaseolin initiation codon.

An AccI site contained in the polylinker region of pTZ19U was then removed from pD-3 by restricting pD-3 with Sall and BamHI, filling-in the ends using the klenow fragment of PolI, isolating the filled-in 4 kb fragment containing the phaseolin sequences, and inserting this fragment into pTZ19U which had been cut with HincII (step not shown in Figure 4). The resulting plasmid, pD3-8, was then digested with AccI, removing the phaseolin coding sequences, the ends were filled-in, and the 5 kb fragment containing the promoter and 3' untranslated regions of the phaseolin gene was gel-purified. Brazil nut sulfur-rich protein cDNA clone pHS-3 was digested with

Hinfl, the ends were filled-in, and the 449 bp fragment containing the 17 kd coding region was gel-purified. The 5 kb fragment containing the phaseolin regulatory sequences and the 449 bp fragment containing the sulfur-rich protein coding sequences were ligated together and transformed into NM522 cells. The resulting clones were screened by restriction analysis for those with the coding sequence in the proper orientation and the junctions between the phaseolin regions and the Brazil nut regions were sequenced. The clone pD3-8-12 contains the phaseolin promoter attached to Brazil nut sequences encoding the 17 kd precursor of the sulfur-rich protein cDNA and the 3' untranslated phaseolin sequences of phaseolin.

E. coli NM522 (pD3-8-12) was deposited on 19 June 1987 with the American Type Culture Collection, 12301 Parklawn Dr., Rockville, MD 20852, and assigned accession number 67445.

## IV. Construction of the 15 kd, 12 kd and 9 kd Phaseolin-Brazil Nut Fusion Proteins

The construction of additional expression cassettes encoding Brazil nut storage protein subunits is described below and shown schematically in Figure 5.

The plasmid pB-13, which was described for the 17 kd chimeric gene construct, was used in the construction of all three fusion protein genes. For the 15 kd and 9 kd constructs, a Smal site was inserted in pB-13 in the phaseolin sequence just beyond the presumed signal peptide cleavage site by site-directed mutagenesis using the oligonucleotide 5'-CCTCCTCCCGGGGGTGAAG-3'. An Accl site and a Smal site contained in the polylinker region of the vector pTZ19U were removed from the resulting plasmid, pE-7, in a manner analogous to that described in the construction of the 17 kb construct (not shown in Fig. 5), resulting in the plasmid pE7-52-1. Next, pE7-52-1 was digested with Smal and Accl to remove the phaseolin coding sequences, the ends were filled-in using the Klenow fragment of PolI, and the 5 kb fragment containing the phaseolin promoter, signal peptide and 3' untranslated region was gel-purified. The Brazil nut cDNA clone pHS-3 was digested with either Rsal and Hinfl (for the 9 kd construct) or Bgll and Hinfl (for the 15 kd construct), the ends made blunt, and the 247 bp Rsal-Hinfl fragment (for the 9 kd construct) and the 372 bp Bgll-Hinfl fragment (for the 15 kd construct) were gel-purified. These Brazil nut fragments were then ligated to the 5 kb phaseolin fragment and transformed into NM522 cells. The clones pRsa-15 (9 kd construct) and pBgl-32 (15 kd construct) contain the Brazil nut sequences in the proper orientations.

For the construction of the 12 kd chimeric gene, an Xhol site was inserted in pB-13 in the phaseolin gene just beyond the presumed signal peptide cleavage site by site-directed mutagenesis using the oligonucleotide 5'-CTCCTCCTCGAGTGAAGT-3', resulting in the plasmid pF-11. Once again, the Accl site found in the pTZ19U portion of pF-11 was removed, generating the plasmid, pF11-50 (step not shown in Fig. 5). pF11-50 was then digested with Xhol and Accl to remove the phaseolin coding sequences, the ends were filled-in, and the 5 kb fragment containing the phaseolin promoter, signal peptide and 3' untranslated region was gel-purified. Brazil nut sulfur-rich protein cDNA clone pHS-3 was digested with Thal and Hinfl, the ends were filled-in, and the 321 bp fragment encoding the 12 kd region of the sulfur-rich protein was gel purified. This Brazil nut fragment was then ligated to the 5 kb phaseolin fragment and transformed into NM522 cells. Clone pTha-18 contains the Brazil nut insert in the proper orientation.

The junctions between the phaseolin regions and the Brazil nut regions of pRsa-15, pBgl-32 and pTha-18 were sequenced to ensure that the coding regions from the two proteins were fused in the proper reading frame.

## V. Transfer of the Phaseolin-Brazil Nut Chimeric Genes to Tobacco Plants

The chimeric genes were digested with either HindIII or EcoRI and inserted into the plasmid pARC12, a derivative of pARC8. Simpson et al. (1986) Plant Mol. Biol. 6:403 whose construction is shown in Figure 7. This plasmid is part of a binary Ti plasmid vector system of Agrobacterium tumefaciens and contains the chimeric gene nopaline synthase/neomycin phosphotransferase (NOS/NPT) as a selectable maker for transformed plant cells. The vectors containing the chimeric Brazil nut genes were transferred by conjugation to Agrobacterium strain LBA4404 [Hockema et al. (1983) Nature 303:179-181] and the resulting bacteria were used to inoculate tobacco leaf discs. Plants were regenerated in selective medium containing kanamycin. Horsch et al. (1985) Science 227:1229-1231.

## IV. Transformation of Alfalfa Plants with Chimeric Brazil Nut Genes

The pARC12 vectors containing the chimeric Brazil nut gene were first transferred by conjugation into Agrobacterium rhizogenes strain A4 [White et al. (1980) J. Bacteriol. 141:1134], and the resulting bacteria were used to inoculate stem segments of mature alfalfa plants (Medicago sativa L.) cv. CUF 101. After 2-3 weeks incubation on TM-1 solid medium, hairy roots were induced on these stem segments. These roots were excised and cultured on hormone-free TM-4 medium for further growth. Shahin (1985) Theor. Appl. Genet. 69:236. NPT assay [Reiss et al. (1984) Gene 30:211] was used to monitor transformation. The hairy roots were transferred to callus induction medium. The hairy root-derived calli were subsequently moved onto LB-3 and then TM-40 media for embryoid development. Regeneration of alfalfa plants could be achieved by continuous subculturing of the embryoids on TM-1 medium or on BOiY2 medium of Bingham et al. (1976) Crop Science 15:719, as reported by Sukhapinda et al. (1987) Plant Mol. Biol. 8:209.

Variations on the above embodiments are readily within the skill of the ordinary artisan and do not depart from the scope of the present invention as described in the following claims.

# Claims

1. A DNA molecule comprising an expression cassette containing a coding sequence encoding at least one subunit of the sulfur-rich 2S seed storage protein of Bertholletia excelsa H.B.K. under the control of regulatory sequences that provide for the transcription of said coding sequence in a cell, the promoter of said regulatory sequences being heterologous to said coding sequence.

2. A DNA molecule comprising an expression cassette containing a coding sequence encoding at least one subunit of the sulfur-rich 2S seed storage protein of Bertholletia excelsa H.B.K. under the control of regulatory sequences that provide for the transcription of said coding sequence in a cell, said coding sequence also encoding a signal sequence heterologous to said subunit.

3. A cell comprising a replicon containing an expression cassette comprised of a DNA coding sequence encoding at least one subunit of the sulfur-rich 2S seed storage protein of Bertholletia excelsa H.B.K. under the control of DNA regulatory sequences that provide for the transcription of said coding sequence in said cell, said subunit being heterologous to said cell.

4. A cell as claimed in Claim 3 wherein the promoter of said regulatory sequences is not said 2S seed storage protein promoter.

5. A cell as claimed in Claim 3 or Claim 4 and which is a higher plant cell.

6. A cell as claimed in any one of Claims 3 to 5 wherein said subunit is expressed fused to a heterologous signal sequence.

7. A DNA molecule as claimed in Claim 1 or a cell as claimed in any one of Claims 3 to 6 wherein said promoter or the promoter of said regulatory sequences, as the case may be, is a seed storage protein promoter.

8. A DNA molecule as claimed in Claim 2 or a cell as claimed in Claim 6 wherein said signal sequence is a seed storage protein signal sequence.

9. A DNA molecule or cell as claimed in Claim 7 or Claim 8 wherein said seed storage protein is phaseolin.

10. A DNA molecule as claimed in any one of Claims 1, 2, or 7 to 9, or a cell as claimed in any one of Claims 3 to 9, wherein said coding sequence encodes:-

    i. the 17 kd prepropeptide of said storage protein; or

    ii. the 15 kd propeptide of said storage protein; or

    iii. the 12 kd propeptide of said storage protein; or

    iv. the 9 kd subunit of said storage protein; or

    v. the 3kd subunit of said storage protein.

11. A cell comprising a replicon containing an expressible DNA coding sequence encoding for a sulfur-rich 2S seed storage polypeptide of Bertholletia excelsa H.B.K. and as obtainable from ATCC 67445, or plasmid pD3-8-12 obtainable from ATCC 67445.

12. Plant tissue comprising cells as claimed in any one of Claims 3 to 10, or a plant comprising such tissue.

13. A seed comprising plant tissue as claimed in Claim 12, e.g. a legume seed.

14. A plant seed other than of Bertholletia excelsa H.B.K., comprising the sulfur-rich 2S storage protein of Bertholletia excelsa H.B.K.

15. A method of producing a DNA molecule comprising an expression cassette containing a coding sequence encoding at least one subunit of the sulfur-rich 2S seed storage protein of Bertholletia excelsa H.B.K., which method comprises ligating to said coding sequence regulatory sequences that provide for the transcription of said coding sequence in a cell, the promoter of said regulatory sequences being heterologous to said coding sequence and/or a signal sequence encoded by said coding sequence being heterologous to said subunit.

16. A method of making a cell having expressible DNA encoding at least one subunit of the sulfur-rich 2S seed storage protein of Bertholletia excelsa H.B.K., which method comprises (a) incorporating into the expressible genome of a cell DNA as defined in any one of Claims 1, 2 or 7 to 10; or, (b) incorporating into the cell a DNA coding sequence encoding at least one subunit of the sulfur-rich 2S seed storage protein of Bertholletia excelsa H.B.K. under the control of DNA regulatory sequences that provide for the transcription of said coding sequence in said cell, said subunit being heterologous to said cell.

17. A process for the production of a plant containing the sulfur-rich 2S seed storage protein of Bertholletia excelsa H.B.K. comprising introducing into the expressible genome of selected plant tissue other than Bertholletia excelsa H.B.K., a DNA coding sequence encoding at least one subunit of the sulfur-rich 2S seed storage protein of Bertholletia excelsa H.B.K. under the control of DNA regulatory sequences that provide for the transcription of said coding sequence in cells of said tissue and culturing the resulting engineered tissue.

18. A foodstuff or foodstuff ingredient comprising tissue/cells other than Bertholletia excelsa H.B.K. enriched with a sulfur-rich 2S seed storage polypeptide of Bertholletia excelsa H.B.K.

19. A process for providing a foodstuff or foodstuff ingredient as defined in Claim 18, which comprises engineering cells other than Bertholletia excelsa H.B.K. to express at least one subunit of the sulfur-rich 2S seed storage protein of <u>Bertholletia excelsa</u> H.B.K. and cultivating said cells.